Europäisches Patentamt

European Patent Office

Office européen des brevets

(11) Publication number: **0 284 765 B1**

## EUROPEAN PATENT SPECIFICATION

(45) Date of publication of patent specification: **18.05.94**  (51) Int. Cl.5: **A61K 7/32**

(21) Application number: **88102480.6**

(22) Date of filing: **19.02.88**

(54) **Soap based gel sticks.**

(30) Priority: **04.03.87 US 21849**

(43) Date of publication of application:
**05.10.88 Bulletin  88/40**

(45) Publication of the grant of the patent:
**18.05.94 Bulletin  94/20**

(84) Designated Contracting States:
**AT BE CH DE ES FR GB GR IT LI LU NL SE**

(56) References cited:
**EP-A- 0 013 390        EP-A- 0 089 120
EP-A- 0 107 330        DE-B- 1 046 262
DE-B- 1 265 344        GB-A- 2 062 466**

(73) Proprietor: **THE PROCTER & GAMBLE COM-
PANY
One Procter & Gamble Plaza
Cincinnati Ohio 45202(US)**

(72) Inventor: **Mortillo, Susan M.
54 Pheasant Run
Edison, NJ(US)**
Inventor: **Hill, John H.
50 Pheasant Run
Edison, NJ(US)**

(74) Representative: **Wächtershäuser, Günter, Dr.
Tal 29
D-80331 München (DE)**

Rank Xerox (UK) Business Services
(3.10/3.09/3.3.3)

EP 0 284 765 B1

**Description**

This invention relates to cosmetic stick products. More particularly the invention relates to a cosmetic stick product that is a clear, translucent gel based on a long chain fatty soap and which is aesthetically pleasing when applied to the skin and has improved formula stability.

The cosmetic stick is one of the oldest systems for delivering dyes, pigments, deodorants, astringents and numerous other beneficiating agents to the human body. One of the long established applications for sticks has been personal deodorants. Although at one time sticks constituted a small part of the total deodorant market, the decline of fluorocarbon propelled aerosol deodorants and the sticks convenience over creams and liquids have resulted in an increase in stick use.

The traditional stick deodorant is based on sodium stearate, often made by the neutralization of stearic acid in ethanol solution with sodium hydroxide. A perfume, bacteriostat and humectant can be added, and other solvents other than ethanol can be used, including water. Some current commercial sticks use propylene glycol. Overall, the function of a deodorant stick is accomplished by inhibiting the growth of odor causing bacteria with a bacteriostat and by use of a relatively large concentration of fragrance to mask body odor.

It will be seen that the functional effectiveness of the stick is of primary importance. However, also of great importance in consumer acceptance of the product are the aesthetic properties and stability. The product should have a pleasing appearance, it should have a pleasant odor. It should feel good when being applied to the skin, i.e. it should not be dry, gritty, greasy, oily, sticky and the like. It should not dry out, shrink or change color over a period of time. Some of these aesthetic properties might not change the functionality of the stick, but the consumer given a choice of a more esthetically pleasing stick will probably choose it even though functionally it is no more effective than a stick having less aesthetic characteristics.

The current known sticks based on sodium stearate and ethanol have suffered from stability problems such as internal and external crystal formation and syneresis. The sticks are usually opaque in appearance to minimize consumer perception of these problems. Moreover, the ethanol solvent volatilized from such sticks. The result is that the stick becomes dry and hard and also shrinks away from the inner surface of the container. Attempts were made to solve this problem by using propylene glycol as the solvent. However, this solvent also causes instability in the stick formulation resulting in crystal formulation and syneresis. European Patent Application 13,390 discloses a solid stick-type cosmetic composition containing sodium stearate which may also contain, among other integrients, a polyhydroxyl compound, such as glycol or a polyglycol in an amount of from about 0.5 to 10% by weight. British patent application GB 2,062,466 discloses an anti-perspirant composition containing dibenzaldehyde-monosorbitol acetal which reduces stickiness by limitting the proportion of propylene-ethylene glycol polycondensate present and/or by incorporating one or more oleaginous compounds. The compositions comprise by weight 5% to 80% lower monohydric alcohols, 5% to 75% di- and/or polyhydric alcohols and/or polyglycols and/or (poly) glycol ethers having at least one remaining -OH radical, 0.5% to 10% dibenzaldehyde monosorbitol acetal, 2% to 20% antiperspirant metal compound and optionally up to 15% higher fatty acid mono- or di alkanolamide, up to 25% of an oleaginous substance, up to 10% propylene-ethylene glycol polycondensate, and up to 1% higher fatty acid.

The problems of the prior art are solved in accordance with the present invention by providing a clear, translucent gel cosmetic stick composition as defined in Claim 1.

We have found that the problems of the prior art may be overcome, and a stable, aesthetically pleasing, clear, translucent gel stick based on sodium stearate can be made using dipropylene glycol as the solvent. Certain other solvents may form part of the solvent system, i.e. water, propylene glycol or ethanol, so long as dipropylene glycol is present, and so long as the solvent does not contribute any objectionable aesthetic characteristics

Other known ingredients used in deodorant stick products may also be incorporated, such as emollients, humectants, bacteriostats, dyes, pigments, anti-irritants and fragrances. It will also be clear that other ingredients may be applied to the skin, e.g. medicaments by the use of the present stick.

Thus by the use of dipropylene glycol in combination with propylene glycol, a clear, translucent gel cosmetic stick has been obtained that does not exhibit crystallinity or syneresis and is aesthetically pleasing to the user both in appearance and feel to the skin.

The amount of dipropylene glycol varies from 50 to 81 per cent by weight. The amount of propylene glycol is from about 0 to 60 per cent. Water is used in an amount of from about 5 to 20 percent, preferably 5 to 13 per cent. The amount of sodium stearate is from about 6 to 9 per cent. Ethanol is used in an amount of 0 to 66 per cent. The amount of the bacteriostat will vary according to the activity thereof, e.g. triclosan is used up to about 1 per cent. Other ingredients which may be used in the inventive stick are

EP 0 284 765 B1

Carbowax 400, from 0 to 20 per cent; isocetylalcohol from 0 to 5 per cent; Witconol APM from 0 to 20 per cent; sodium metabisulfite from to 0 - 0.1 per cent and disodium ethylene diamine tetraacetic acid from 0 to 0.1 per cent.

The method of formulation will depend on whether sodium stearate is used as the soap base for the stick or whether sodium stearate it is formed in situ from stearic acid and sodium hydroxide.

A number of formulation Examples are shown in Table I. In Examples 1 to 9 and 13 to 16, sodium stearate was used as the base. These formulations are prepared by combining the dipropylene glycol, propylene glycol and triclosan and heating from 80 to 85°C until trichlosan is completely dissolved. If ethanol and water are used, they are also included here. Sodium stearate is then added with continued heating and mixing until the solution is clear. After this, the temperature is lowered to about 75°C and color and fragrance are added (and menthol if it is an ingredient of the formulation). The mixture is then brought down to between 59 to 71°C the pour temperature, which varies depending on the formula ingredients, .

When the sodium stearate is obtained in situ from stearic acid and sodium hydroxide as in Examples 10 to 12, the method of preparation is as follows:

(1) Combine the dipropylene glycol, propylene glycol and triclosan and begin heating and mixing until triclosan is completely dissolved (about 35°C);

(2) Add the stearic acid and heat until dissolved (52°C);

(3) Dissolve the sodium hydroxide pellets in batch water and heat to 52°C;

(4) Add the sodium hydroxide solution to vortex of batch;

(5) Mix and heat to clear solution (72°C);

(6) Add fragrance and color (menthol if an ingredient); and

(7) Cool to pouring temperature.

In both methods, the final mixture is poured into the containers and allowed to cool to solidification, thereby forming a solid cosmetic deodorant stick.

It will be understood that various types of containers known in the art may be used and the process steps for pouring and cooling the mixture will vary according to the type of container.

3

EP 0 284 765 B1

TABLE I

| Example | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| Dipropylene glycol | 60.0 | 21.0 | 50.0 | 40.0 | 50.0 | --- | 60.0 | 60.0 | 21.0 | 60.0 | 60.0 | 81 | 60.0 | 60.0 | 60.0 |
| Propylene glycol | 21.0 | 50.0 | 21.0 | 20.0 | 21.0 | 40.0 | --- | 21.0 | 50.0 | 21.0 | 21.0 | 0 | 21.0 | 21.0 | 21.0 |
| Water | 9.7 | 9.7 | 13.6 | 20.0 | 9.7 | 19.8 | 19.8 | 7.7 | 9.6 | 8.7485 | 9.50 | 7.7 | 9.69 | 9.25 | 9.19 |
| Sodium Stearate | 8.0 | 8.0 | 8.0 | 7.5 | 8.0 | 7.9 | 7.5 | 8.0 | 8.0 | | | 8.0 | 8.0 | 8.0 | 8.0 |
| Carbowax 400 | | 10.0 | | | | | | | 10.0 | | | | | | |
| Isocetyl Alcohol | | | 5.0 | | | | | | | | | | | | |
| Witconol APM | | | | 10.0 | | | | | | | | | | | |
| Ethanol | | | | | 10.0 | | | | | | | | | | |
| Carbitol | | | | | | 30.0 | | | | | | | | | |
| 1, 3 Butylene glycol | | | | | | | 11.0 | | | | | | | | |
| Benzyl Alcohol | | | | | | | | 2.0 | | | | | | | |
| Menthol | | | | | | | | | 0.10 | | | | | | |
| Miscellaneous (Stability Agent, Fragrance, Color) | 1.05 | 1.05 | 2.15 | 2.25 | 1.05 | 2.05 | 1.45 | 1.05 | 1.05 | 1.505 | 0.75 | 1.3 | 1.06 | 1.5 | 1.56 |
| Stearic Acid | | | | | | | | | | 7.4 | 7.4 | | | | |
| Sodium hydroxide | | | | | | | | | | 1.1 | 1.1 | | | | |
| Triclosan | 0.25 | 0.25 | 0.25 | 0.25 | 0.25 | 0.25 | 0.25 | 0.25 | 0.25 | 0.25 | 0.25 | 0.25 | 0.25 | 0.25 | 0.25 |
| Oleic Acid | | | | | | | | | | * | | | | | |

* to adjust free fatty acids to 0.05 - 0.17%

1. Carbowax 400 is a medium molecular weight polyoxyethylene polymer of Union Carbide Company.
2. Witkonol APM is a polyoxyethylene glycol myrystyl ether of Witco Chemicals.

Examples 2, 4 and 9 are given for illustrative purposes.

Example 16

In this Example, a comparison is made between the deodorant stick according to the formulation of Example 13 in Table I to show superior deodorant efficacy against commercial stick deodorants (1)

4

Commercial Ethanol Stick #1; (2) Commercial Propyleneglycol Stick and (3) Commercial Ethanol Stick #2. In each comparison 60 males were used. The comparisons were (1) Example 13 vs. Commercial Ethanol Stick #1; (2) Example 13 vs. Commercial Propyleneglycol Stick and (3) Example 13 vs. Commercial Ethanol Stick #2. Subjects were evaluated for axillary odor approximately 24 hours after one, two and three product applications. The results, taken after the third treatment are shown in Table II.

Table II shows that the deodorant stick of the present invention is highly efficacious and has unexpectedly superior efficacy over known commercial deodorants.

## Table II

| Product | Mean Odor Score |
|---|---|
| Example 13 | 1.0 |
| Commercial Ethanol Stick # 1 | 1.7 |
| Example 13 | 0.8 |
| Commercial Propyleneglycol Stick | 1.4 |
| Example 13 | 0.7 |
| Commercial Ethanol Stick # 2 | 1.0 |

## Claims

1. A clear, translucent gel cosmetic stick composition comprising dipropylene glycol and a long chain fatty acid soap of the type sodium stearate, wherein the dipropylene glycol comprises 50-81% by weight of the composition.

2. The stick composition of claim 1 wherein said soap is sodium stearate which is present from about 6% to about 9% by weight of the composition.

3. The stick of claim 2 wherein said sodium stearate is formed in situ from stearic acid and sodium hydroxide.

4. The stick composition of claim 1 including propylene glycol.

5. The stick composition of claim 1 including ethanol.

6. The stick composition of claim 1 including water.

7. The stick composition of claim 1 including a deodorant compound.

8. The stick composition of Claim 7 wherein said deodorant compound is a bacteriostat.

**Patentansprüche**

1.  Klares durchscheinendes gelförmiges kosmetisches stiftartiges Mittel, umfassend Dipropylenglycol und eine langkettige Fettsäureseife vom Natriumstearattyp, wobei das Dipropylenglycol 50-81 Gew.-% des Mittels umfaßt.

2.  Stiftartiges Mittel nach Anspruch 1, wobei die Seife Natriumstearat ist, die in einer Menge von etwa 6 bis etwa 9 Gew.-% des Mittels vorliegt.

3.  Stift nach Anspruch 2, wobei das Natriumstearat in situ aus Stearinsäure und Natriumhydroxid gebildet wird.

4.  Stiftartiges Mittel nach Anspruch 1, Propylenglycol einschließend.

5.  Stiftartiges Mittel nach Anspruch 1, Ethanol einschließend.

6.  Stiftartiges Mittel nach Anspruch 1, Wasser einschließend.

7.  Stiftartiges Mittel nach Anspruch 1, eine desodorierende Verbindung einschließend.

8.  Stiftartiges Mittel nach Anspruch 7, wobei die desodorierende Verbindung ein bakteriostates Mittel ist.

**Revendications**

1.  Composition pour bâton cosmétique gélifié, limpide et transparent comprenant du dipropylèneglycol et un savon à base d'acide gras à longue chaîne du type stéarate de sodium, dans laquelle le dipropylèneglycol constitue de 50 à 81% en poids de la composition.

2.  Composition pour bâton selon la revendication 1, dans laquelle ledit savon est le stéarate de sodium, qui est présent d'environ 6% à environ 9% en poids de la composition.

3.  Bâton selon la revendication 2, dans lequel ledit stéarate de sodium est formé in situ à partir d'acide stéarique et d'hydroxyde de sodium.

4.  Composition pour bâton selon la revendication 1 incluant du propylèneglycol.

5.  Composition pour bâton selon la revendication 1 incluant de l'éthanol.

6.  Composition pour bâton selon la revendication 1 incluant de l'eau.

7.  Composition pour bâton selon la revendication 1 incluant un composé déodorant.

8.  Composition pour bâton selon la revendication 7, dans laquelle ledit composé déodorant est un agent bactériostatique.